# DEMANDE DE BREVET EUROPEEN

(11) **EP 3 564 343 A1**
(43) Date de publication de la demande: **06.11.2019**
(21) Numéro de dépôt: 19169112.0
(22) Date de dépôt: 12.04.2019
(51) Int. Cl.: C10G 7/06, C07C 5/27, C07C 7/00, C07C 7/04, C07C 7/10, C07C 7/12

(54) **PROCEDE ET DISPOSITIF DE SEPARATION D'AROMATIQUES SOUS VIDE**

(30) Priorité: 04.05.2018 FR 1853884
(71) Demandeur: AXENS, 92508 Rueil-Malmaison Cedex (FR)
(72) Inventeur: COTTE, Arnaud, 92000 NANTERRE (FR); PIGOURIER, Jérôme, 92190 MEUDON (FR); PUPAT, Nicolas, 78800 HOUILLES (FR)
(74) Mandataire: IFP Energies nouvelles

(57) **Abrégé**

La présente invention concerne un procédé et un dispositif de séparation d'une charge comprenant du benzène, du toluène et des composés C8+, au moyen d'au moins une colonne à distiller dite de reformat (C1), une unité d'extraction des aromatiques (P1), une unité de séparation du paraxylène (P2), une unité d'isomérisation des xylènes (P3) et une unité de transalkylation (P4), les effluents desdites unités étant séparés dans les colonnes à distiller suivantes : colonne de purification (C6), déheptaniseur (C7) et colonne de toluène (C10), dans lesquels au moins une desdites colonnes à distiller est adaptée pour être opérée sous vide de sorte que : la majorité des composés C7- sont récupérés dans le produit de tête de la colonne à distiller opérée sous vide, et la majorité des composés C8+ sont récupérés dans le produit de fond de la colonne à distiller opérée sous vide.

## Description

### Domaine technique

La présente invention s'inscrit dans le domaine des procédés et dispositifs de séparation de composés aromatiques. L'objet de la présente invention s'applique en particulier au cas d'un complexe aromatique faisant appel à une séparation par distillation entre du benzène et/ou du toluène et des composés plus lourds à 8 atomes de carbones ou plus (notés ci-après composés C8+).

### Etat de l'art

Le brevet FR2998301B1 décrit une méthode permettant un gain énergétique global sur la consommation de combustible et d'électricité d'un complexe aromatique pour la séparation par distillation entre du benzène, du toluène et des composés C8+. Spécifiquement, le principe de ladite méthode réside dans la récupération de la chaleur considérée comme perdue en raison de son faible niveau de température (autour de 100°C à 180°C) en générant de la vapeur basse pression au niveau de certaines colonnes, la vapeur basse pression ainsi générée étant utilisée comme fluide caloporteur dans les rebouilleurs de certaines colonnes et/ou certains préchauffeurs utilisés dans ladite méthode. Ceci permet pour un complexe aromatique, un gain énergétique global substantiel sur la consommation de combustible et d'électricité.

Un inconvénient du brevet FR2998301B1 est que la recompression de la vapeur basse pression avant utilisation comme fluide caloporteur dans les rebouilleurs est requise sur l'immense majorité des colonnes. En effet, selon la Table 2 du brevet FR2998301B1, cinq colonnes utilisent la vapeur basse pression produite à l'intérieur du complexe mais uniquement le stripeur le fait sans recompression.

### Résumé

Dans le contexte précédemment décrit, un premier objet de la présente invention est de permettre la réduction de la quantité d'énergie requise pour réaliser la séparation entre du benzène et/ou du toluène et des composés C8+. Spécifiquement, le premier objet consiste à opérer sous vide des colonnes d'un complexe aromatique habituellement opérées sous pression. Selon un ou plusieurs modes de réalisation, il est possible de procéder à un échange direct entre les colonnes productrices et consommatrices d'énergie basse température. L'échange direct peut-être substitué par une génération de vapeur d'eau basse pression par les colonnes productrices de chaleur basse température et utilisation par les colonnes opérées sous vide, sans recompression de cette vapeur d'eau.

Selon un premier aspect, l'objet précité, ainsi que d'autres avantages, sont obtenus par un procédé de séparation d'une charge comprenant du benzène et/ou du toluène et des composés à 8 atomes de carbone ou plus, dans un dispositif de séparation comprenant au moins une colonne à distiller dite de reformat, une unité d'extraction des aromatiques, une unité de séparation du paraxylène, une unité d'isomérisation des xylènes et une unité de transalkylation, les effluents desdites unités étant séparés dans les colonnes à distiller suivantes : colonne de purification, déheptaniseur et colonne de toluène,
dans lequel on opère sous vide au moins une desdites colonnes à distiller de sorte que :
la majorité des composés à 7 atomes de carbones ou moins sont récupérés dans le produit de tête de la colonne à distiller opérée sous vide, et
la majorité des composés à 8 atomes de carbone ou plus sont récupérés dans le produit de fond de la colonne à distiller opérée sous vide.

Selon un ou plusieurs modes de réalisation, le produit de fond de la colonne à distiller opérée sous vide a une teneur supérieure à 25% poids (de façon préférée 35% poids et encore plus préférée 50% poids) en composés à 8 atomes de carbones et/ou composés dont la température normale d'ébullition est inférieure à 150°C.

Selon un ou plusieurs modes de réalisation, la pression en tête de colonne de la colonne à distiller opérée sous vide est comprise entre 0,03 MPa et 0,095 MPa (de façon préférée entre 0,04 MPa et 0,085 MPa et encore plus préférée entre 0,05 et 0,075 MPa).

Selon un ou plusieurs modes de réalisation, le rebouilleur de la colonne à distiller opérée sous vide est opéré à une température inférieure à 180°C (de façon préférée inférieure à 165°C et encore plus préférée inférieure à 150°C).

Selon un ou plusieurs modes de réalisation, une chaleur basse température est apportée au rebouilleur de la colonne à distiller opérée sous vide à partir d'énergie basse température disponible dans le dispositif de séparation, soit par échange direct, soit par l'intermédiaire de génération de vapeur d'eau utilisée sans recompression.

Selon un ou plusieurs modes de réalisation, la teneur en C4- dans le produit de tête de la colonne à distiller opérée sous vide est inférieure à 1% mol (de façon préférée inférieure à 0,5% mol et encore plus préférée inférieure à 0,1% mol).

Selon un ou plusieurs modes de réalisation, le produit de tête de la colonne à distiller opérée sous vide est dirigé vers une colonne additionnelle comportant en tête de colonne un flux vapeur purgé en dehors du procédé de séparation (de façon préférée pour purger de l'oxygène avec le flux vapeur purgé en tête de colonne de la colonne additionnelle).

Selon un ou plusieurs modes de réalisation, on opère le rebouilleur de la colonne additionnelle à une température inférieure à 180°C (de façon préférée par échange direct ou en utilisant un fluide caloporteur intermédiaire ne nécessitant pas de recompression).

Selon un deuxième aspect, l'objet précité, ainsi que d'autres avantages, sont obtenus par un dispositif de séparation d'une charge comprenant du benzène, du toluène et des composés à 8 atomes de carbone ou plus, comprenant au moins une colonne à distiller dite de reformat, une unité d'extraction des aromatiques, une unité de séparation du paraxylène, une unité d'isomérisation des xylènes et une unité de transalkylation, le dispositif de séparation comprenant en outre les colonnes suivantes pour la distillation des effluents desdites unités : colonne de purification, déheptaniseur et colonne de toluène,
dans lequel au moins une desdites colonnes à distiller est adaptée pour être opérée sous vide de sorte que :
la majorité des composés à 7 atomes de carbones ou moins sont récupérés dans le produit de tête de la colonne à distiller opérée sous vide, et
la majorité des composés à 8 atomes de carbone ou plus sont récupérés dans le produit de fond de la colonne à distiller opérée sous vide.

Selon un ou plusieurs modes de réalisation, l'au moins une desdites colonnes à distiller est adaptée de sorte que le produit de fond de la colonne à distiller opérée sous vide ait une teneur supérieure à 25% poids (de façon préférée 35% poids et encore plus préférée 50% poids) en composés à 8 atomes de carbones et/ou composés dont la température normale d'ébullition est inférieure à 150°C.

Selon un ou plusieurs modes de réalisation, l'au moins une desdites colonnes à distiller est adaptée de sorte que la pression en tête de colonne de la colonne à distiller opérée sous vide soit comprise entre 0,03 MPa et 0,095 MPa (de façon préférée entre 0,04 MPa et 0,085 MPa et encore plus préférée entre 0,05 et 0,075 MPa).

Selon un ou plusieurs modes de réalisation, l'au moins une desdites colonnes à distiller est adaptée de sorte que le rebouilleur de la colonne à distiller opérée sous vide soit opéré à une température inférieure à 180°C (de façon préférée inférieure à 165°C et encore plus préférée inférieure à 150°C).
Selon un ou plusieurs modes de réalisation, l'au moins une desdites colonnes à distiller est adaptée de sorte le rebouilleur de la colonne à distiller opérée sous vide puisse être alimenté par une chaleur basse température provenant d'énergie basse température disponible dans le complexe, soit par échange direct, soit par l'intermédiaire de génération de vapeur d'eau utilisée sans recompression.

Selon un ou plusieurs modes de réalisation, l'au moins une desdites colonnes à distiller est adaptée de sorte que la teneur en C4- (composés à 4 atomes de carbones ou moins) dans le produit de tête de la colonne à distiller opérée sous vide soit inférieure à 1% mol (de façon préférée inférieure à 0,5% mol et encore plus préférée inférieure à 0,1% mol).

Selon un ou plusieurs modes de réalisation, le dispositif de séparation comprend en outre une colonne additionnelle, en aval de la sortie de tête de la colonne à distiller opérée sous vide, pour purger un flux vapeur en tête de colonne en dehors du dispositif de séparation (de façon préférée pour purger de l'oxygène avec le flux vapeur purgé en tête de colonne de la colonne additionnelle).

Selon un ou plusieurs modes de réalisation, la colonne additionnelle est adaptée de sorte que le rebouilleur de la colonne additionnelle soit opéré à une température inférieure à 180°C (de façon préférée par échange direct ou en utilisant un fluide caloporteur intermédiaire ne nécessitant pas de recompression).

Des modes de réalisation du procédé et du dispositif référencés ci-dessus ainsi que d'autres caractéristiques et avantages vont apparaître à la lecture de la description qui va suivre, donnée à titre uniquement illustratif et non limitatif, et en référence au dessin suivant.

### Brève description du dessin

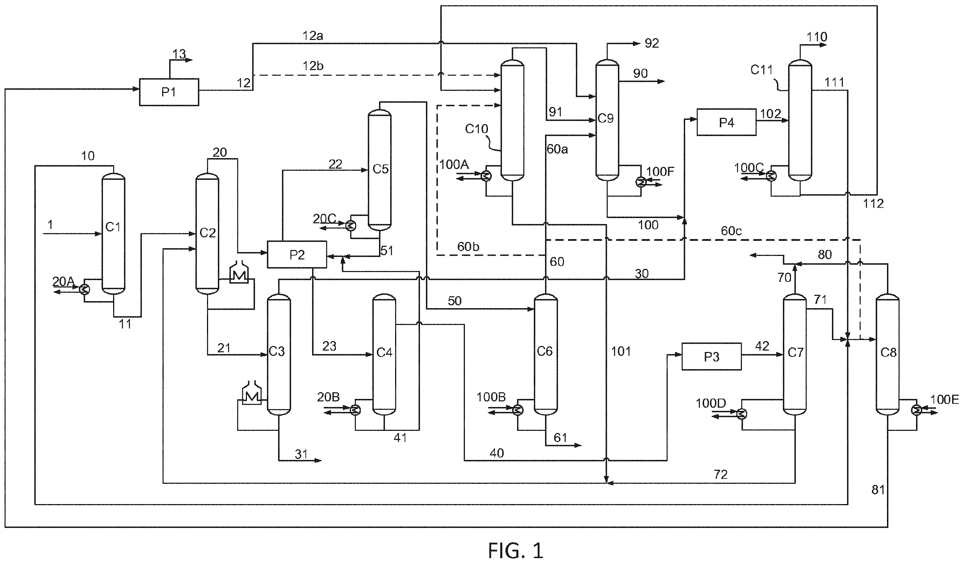

La figure 1 décrit un schéma d'un complexe aromatique selon la présente invention pour la séparation de benzène et/ou de toluène et de composés C8+ dans lequel on opère sous vide au moins une des colonnes à distiller.

### Description détaillée

La présente invention concerne le domaine des procédés et dispositifs pour la séparation d'une charge comprenant du benzène et/ou du toluène et des composés C8+ (e.g. C8 à C10) pouvant notamment comprendre du paraxylène.

Le procédé et dispositif de séparation selon la présente invention peut être défini comme une série d'étapes et sections de conversion et de séparation destinée à séparer benzène et toluène de composés C8+ et notamment des composés aromatiques à huit atomes de carbone, appelé xylènes, et plus particulièrement le paraxylène, à partir d'une charge riche en composés aromatiques allant du benzène à des composés aromatiques à plus de 10 atomes de carbone (notés C10+), provenant par exemple d'une unité de reformage catalytique. La charge riche en composés aromatiques présente typiquement des teneurs en composés soufrés, azotés et oléfines très faible à nulle (e.g. teneur en soufre < 0,5 ppm poids et/ou teneur en azote < 0,5 ppm poids et/ou indice de brome (bromine number en anglais) < 1g/100g selon ASTM D1159), car ces composés peuvent affecter les performances et la durée de vie de certains catalyseurs et des tamis moléculaires mis en oeuvre dans les unités du complexe aromatique.

Le premier objet de la présente invention peut être défini comme l'opération sous vide de colonnes à distiller de sorte que :
- la majorité des composés à 8 atomes de carbone (e.g. xylènes) ou plus sont récupérés dans le produit de fond, et
- la majorité des composés aromatiques à 7 atomes de carbones (e.g. toluène) ou moins (e.g. benzène) sont récupérés dans le produit de tête.

Selon un ou plusieurs modes de réalisation, la teneur en composés à 8 atomes de carbones (ou composés dont la température normale d'ébullition est inférieure à 150°C) dans le produit de fond est supérieure à 25% poids (de façon préférée 35% poids et encore plus préférée 50% poids).

Selon un ou plusieurs modes de réalisation, la teneur en C4- dans le produit de tête est inférieure à 1% mol (de façon encore plus préférée 0,5% mol et encore plus préférée 0,1% mol).

Une telle colonne à distiller permet si elle opérée sous vide (e.g. à une pression inférieure à la pression atmosphérique) :
- d'avoir une température de rebouilleur en fond inférieure à 180°C (préférablement inférieure à 165°C et de façon plus préférée inférieure à 150°C) car une forte fraction du produit de fond est constituée de composés dont la température normale d'ébullition est inférieure à 150°C ; et
- de pouvoir condenser en tête de ladite colonne à distiller opérée sous vide, la quasi-totalité de la coupe C5-C7 à des températures compatibles avec l'eau de refroidissement disponible en raffinerie. En effet le plus léger des C5+, l'isopentane, présente une température normale d'ébullition égale à 28°C et un point de bulle de 20°C à 0,08 MPa.

D'autre part, lorsque la teneur en légers dans le produit de tête (C4-) est faible (teneur en C4-inférieure à 1% mol, de façon encore plus préférée 0,5% mol et encore plus préférée 0,1% mol), la colonne à distiller opérée sous vide peut être opérée sous vide sans générer en tête une phase vapeur importante qui devra être recomprimée par un compresseur. Selon un ou plusieurs modes de réalisation, seul un faible débit d'incondensables, notamment ceux liés à une possible entrée d'air dans un système sous vide, est à reprendre par des moyens usuels sur une colonne à distiller sous vide, à savoir un éjecteur ou une pompe à vide.

Selon un ou plusieurs modes de réalisation, la pression en tête de la colonne à distiller opérée sous vide (au niveau de la tubulure de tête de la virole de ladite colonne) est comprise entre 0,03 MPa et 0,095 MPa, préférablement entre 0,04 MPa et 0,085 MPa, et de façon encore plus préférée entre 0,05 et 0,075 MPa.

Selon un ou plusieurs modes de réalisation, la colonne à distiller opérée sous vide est rebouillie par de l'énergie de faible niveau thermique (e.g. à une température inférieure à 180°C) disponible dans le complexe. Ladite colonne pourra par exemple être rebouillie soit directement par la source de chaleur basse température (<180°C), soit par l'intermédiaire de vapeur basse pression mais sans nécessiter sa recompression comme dans les procédés de référence.

L'opération d'une colonne à distiller sous vide pouvant impliquer une faible entrée d'air et donc d'oxygène dans le procédé et le dispositif de séparation et la présence d'oxygène pouvant s'avérer néfaste notamment par un possible empoisonnement des catalyseurs en aval, le produit de tête de la colonne à distiller opérée sous vide peut être dirigé vers une colonne additionnelle compotant en tête de colonne un flux vapeur qui sera purgé en dehors du procédé. Selon un ou plusieurs modes de réalisation, une colonne déjà existante dans le procédé et le dispositif de séparation est utilisée pour purger l'oxygène dans le flux vapeur de tête de cette colonne additionnelle. Par exemple, le stripeur peut être utilisé pour purger l'oxygène des produits de tête de la colonne de reformat et/ou du déheptaniseur et/ou de la colonne de purification (e.g. cas ou la colonne de benzène n'est pas utilisée) ; la colonne de benzène peut être utilisée pour purger l'oxygène des produits de tête de la colonne de purification et/ou de la colonne de toluène.

La charge de la colonne additionnelle étant appauvrie en composés en C8+ et étant très majoritairement constituée de C7- (et/ou composés de température normale d'ébullition inférieure à 120°C), la colonne additionnelle peut être rebouillie par de l'énergie de faible niveau thermique (e.g. à une température inférieure à 180°C), par exemple :
- par échange direct ; ou
- en utilisant un fluide caloporteur intermédiaire, tel que de la vapeur basse pression (ne nécessitant pas de recompression).

Dans la description qui suit on parle de *« complexe »* pour désigner tout dispositif de raffinage ou de pétrochimie comportant au moins deux colonnes à distiller. Cette définition est très large et comprend, par exemple, le dispositif de craquage catalytique des essences et le dispositif de production de paraxylène ou de métaxylènes à partir de coupes aromatiques dit *« complexe aromatique ».* La description qui suit et l'exemple qui illustre le procédé et le dispositif de séparation selon la présente invention sont donnés dans le cas d'un complexe aromatique, mais il est bien entendu qu'un complexe aromatique ne constitue qu'un cas d'application et ne limite en aucun cas la portée du procédé et du dispositif de séparation exposés dans la présente description.

La figure 1 décrit un schéma d'un procédé et dispositif de séparation selon un ou plusieurs modes de réalisation de la présente invention, permettant notamment de diminuer la consommation énergétique nécessaire à la séparation entre le benzène, le toluène et les C8+ par rapport au procédé et complexe aromatique de référence.

En référence à la figure 1, il est notamment décrit des zones du procédé et du dispositif de séparation adaptées pour opérer sous vide au moins une desdites colonnes à distiller (C1, C6, C7 et C10) de sorte que la majorité des composés à 7 atomes de carbones ou moins sont récupérés dans le produit de tête de la colonne à distiller opérée sous vide, et la majorité des composés à 8 atomes de carbone ou plus sont récupérés dans le produit de fond de la colonne à distiller opérée sous vide.

Selon un ou plusieurs modes de réalisation, la colonne de reformat et la colonne de toluène sont adaptées pour être opérées sous vide. Selon un ou plusieurs modes de réalisation, la colonne de purification et la colonne de toluène sont adaptées pour être opérées sous vide. Selon un ou plusieurs modes de réalisation, la colonne de reformat, la colonne de purification et la colonne de toluène sont adaptées pour être opérées sous vide. Selon un ou plusieurs modes de réalisation, la colonne de reformat, la colonne de purification, le déheptaniseur et la colonne de toluène sont adaptés pour être opérés sous vide.

Les étapes et sections de conversion et de séparation du procédé et dispositif de séparation de la présente invention sont décrites plus en détails ci-après.

### Colonne de reformat C1

La charge à traiter est envoyée via la ligne 1 vers la première colonne à distiller notée colonne de reformat C1 qui sépare le toluène et les composés plus légers (coupe de composés C7-) des composés plus lourds (coupe de composés C8 à C10+).

Selon un ou plusieurs modes de réalisation, la colonne de reformat C1 est opérée sous vide de façon à avoir une température inférieure à 180°C (e.g. < 165°C) au rebouilleur 20A. Selon un ou plusieurs modes de réalisation, la colonne de reformat C1 est opérée sous vide de façon à avoir une température inférieure à 150°C au rebouilleur 20A. Ainsi, la colonne de reformat C1 peut être rebouillie par de l'énergie basse température (<180°C).

Dans la figure 1, l'effluent de tête de la colonne de reformat C1 est dirigé vers la colonne de stripage C8 (via ligne 10). Selon un ou plusieurs modes de réalisation, la tête de la colonne de reformat C1 effectue ainsi un passage par la colonne de stripage C8 avant de rejoindre l'unité d'extraction des aromatiques P1 (via la ligne 81).

Dans les procédés et complexes aromatiques de référence, l'effluent de tête de la colonne de reformat C1 est dirigé vers l'unité d'extraction des aromatiques P1.

Bien que le bilan énergétique soit moins avantageux, selon un ou plusieurs modes de réalisation, au moins une partie de l'effluent de tête de la colonne de reformat C1 peut être dirigé directement vers l'unité d'extraction des aromatiques P1. Selon un ou plusieurs modes de réalisation, lorsque la colonne de reformat C1 n'est pas opérée sous vide, au moins une partie de l'effluent de tête de la colonne de reformat C1 peut être dirigé directement vers l'unité d'extraction des aromatiques P1. Ces modes de réalisation sont employés par exemple.

Selon un ou plusieurs modes de réalisation, les composés les plus légers, tels que de l'oxygène, sont purgés en tête de la colonne de stripage C8. Le fond de la colonne de stripage C8, préférablement expurgé de l'oxygène, est envoyé vers l'unité d'extraction des aromatiques P1.

### Unité d'extraction des aromatiques P1

Le toluène et le benzène et optionnellement les composés récupérés en fond du stripeur C8 sont envoyés via la ligne 81 à l'unité d'extraction des aromatiques P1.

L'unité d'extraction des aromatiques P1 sépare la coupe C6-C7, essentiellement aromatique, d'un produit comprenant des composés paraffiniques envoyé à l'extérieur du complexe aromatique via la ligne 13. Le solvant utilisé préférentiellement dans l'unité d'extraction des aromatiques P1 est la N-formylmorpholine (NFM).

En référence à la figure 1, la coupe C6-C7 issue de l'unité d'extraction des aromatiques P1 est dirigée via la ligne 12 soit vers la colonne de benzène C9 (ligne 12a), soit afin d'être purifiée de quelques C8 qu'elle pourrait contenir vers la colonne de toluène C10 (12b).

Selon un ou plusieurs modes de réalisation, l'unité d'extraction des aromatiques P1 comprend une unité de distillation extractive.

### Colonne des xylènes C2

Les composés aromatiques C8-C10+ récupérés au fond de la colonne C1 sont envoyés via la ligne 11 vers la colonne des xylènes C2 pour séparer les composés aromatiques en C9 et plus lourds (composés C9+) d'une coupe xylènes comprenant des composés aromatiques en C8 qui alimentent les unités du complexe aromatique situées en aval.

Selon un ou plusieurs modes de réalisation, la pression opératoire en tête de la colonne des xylènes C2 est maintenue à la pression minimale requise (généralement comprise entre 0,7 et 1,2 MPa absolus), qui permet une température de condensation des vapeurs de tête de la colonne de xylènes C2 suffisante pour être utilisée comme fluide caloporteur pour les rebouilleurs (20B et 20C par exemple) de certaines colonnes à distiller (C4 et C5 par exemple).

### Unité de séparation du paraxylène P2

La coupe xylènes, c'est à dire la coupe de composés aromatiques en C8 contenant du paraxylène, du métaxylène de l'orthoxylène et de l'éthylbenzène, est récupérée en tête de la colonne des xylènes C2 et est envoyée via la ligne 20 vers l'unité de séparation du paraxylène P2 qui récupère sélectivement le paraxylène contenu dans ladite coupe xylènes.

Ladite unité de séparation du paraxylène P2 peut être une unité d'adsorption du paraxylène adaptée pour produire un mélange de paraxylène et de désorbant (appelé l'extrait) et un mélange des autres composés C8- aromatiques et de désorbant (appelé le raffinat).

L'adsorbant utilisé est un tamis moléculaire dédié à l'adsorption du paraxylène, c'est à dire qu'il présente une affinité particulièrement élevée envers ce composé.

Un solide adsorbant couramment utilisé est une zéolithe de type faujasite mise en forme avec un liant silicique échangée au baryum ou au potassium. Le désorbant préférentiellement utilisé est le paradiéthylbenzène (PDEB).

Selon un ou plusieurs modes de réalisation, l'unité de séparation du paraxylène P2 comprend une unité de cristallisation du paraxylène, par exemple telle que décrite dans le brevet US 3,467,724.

Selon un ou plusieurs modes de réalisation, l'unité de séparation du paraxylène P2 comprend une combinaison d'une unité d'adsorption du paraxylène et d'une unité de cristallisation telle que décrite par exemple dans le brevet EP-B- 053191.

### Colonne d'extrait C5

Cette colonne est utilisée lorsque l'unité de séparation du paraxylène est du type adsorption du paraxylène. Le flux d'extrait issu de l'unité d'adsorption du paraxylène et contenant le paraxylène et du désorbant est envoyé via la ligne 22 vers la colonne d'extrait C5 qui sépare le paraxylène du désorbant. Le désorbant récupéré au fond de la colonne d'extrait C5 est renvoyé vers l'unité d'adsorption du paraxylène P2 via la ligne 51. Le paraxylène récupéré en tête de la colonne d'extrait C5 est envoyé vers la colonne de purification C6.

Selon un ou plusieurs modes de réalisation, la colonne d'extrait C5 est opérée sous une très légère pression, dans une gamme de pression allant de 0,2 à 0,4 MPa absolus (au ballon de reflux). Ceci de manière à minimiser à la fois la température du rebouilleur 20C et la quantité de chaleur à fournir audit rebouilleur 20C tout en permettant de produire, via la condensation de tête, de l'énergie de bas niveau thermique (e.g. 150°C-180°C).

La condensation des vapeurs de tête de la colonne d'extrait C5 peut ainsi être, pour tout ou partie, réalisée au moyen d'une génération de vapeur basse pression où par échange direct avec le rebouilleur d'une autre colonne.

### Colonne de purification C6

Le flux de tête de la colonne d'extrait C5 est envoyé via la ligne 50 vers la colonne de purification C6 qui sépare le toluène (qui a été partiellement extrait avec le paraxylène) du paraxylène.

Le paraxylène de haute pureté produit est récupéré en fond de la colonne de purification C6 et conduit en tant que produit fini par pompage pour stockage via la ligne 61.

Selon un ou plusieurs modes de réalisation, la colonne de purification C6 est opérée sous vide de façon à avoir une température inférieure à 180°C (e.g. < 165°C) au rebouilleur 100B. Selon un ou plusieurs modes de réalisation, la colonne de purification C6 est opérée sous vide de façon à avoir une température inférieure à 150°C au rebouilleur 100B. Ainsi, la colonne de purification C6 peut être rebouillie par de l'énergie basse température (<180°C).

Selon un ou plusieurs modes de réalisation, le produit de tête de la colonne de purification C6 est dirigé vers la colonne de benzène C9 (ligne 60a) pouvant comporter en tête de colonne un flux vapeur (92), constitué d'incondensables, purgé en dehors du procédé.

Selon un ou plusieurs modes de réalisation, le produit de tête de la colonne de purification C6 peut être dirigé vers la colonne de toluène C10 (ligne 60b) afin de purifier ledit produit de tête de quelques C8 qu'il pourrait contenir. Selon un ou plusieurs modes de réalisation, le produit de tête de la colonne de toluène C10 est envoyé vers la colonne de benzène C9 pouvant comporter en tête de colonne un flux vapeur (92), constitué d'incondensables, purgé en dehors du procédé.

Selon un ou plusieurs modes de réalisation, le produit de tête de la colonne de purification C6 est dirigé vers la colonne de stripage C8 (ligne 60c) pouvant comporter en tête de colonne un flux vapeur, constitué d'incondensables, purgé en dehors du procédé.

### Colonne de raffinat C4

Le raffinat en provenance de l'unité de séparation du paraxylène P2 est envoyé via la ligne 23 vers la colonne de raffinat C4 qui sépare les C8 aromatiques (raffinat) du désorbant. Le désorbant récupéré en fond de la colonne de raffinat C4 est renvoyé vers l'unité de séparation du paraxylène P2 via la ligne 41.

Le raffinat (coupe C8 aromatiques) est extrait par un soutirage latéral et envoyé via la ligne 40 comme charge de l'unité d'isomérisation des xylènes P3.

Selon un ou plusieurs modes de réalisation, la colonne de raffinat C4 est opérée sous une très légère pression, dans une gamme de pression allant de 0,2 à 0,4 MPa absolus (au ballon de reflux). Ceci de manière à minimiser à la fois la température du rebouilleur 20B et la quantité de chaleur à fournir audit rebouilleur 20B tout en permettant de produire, via la condensation de tête, de l'énergie de bas niveau thermique (150°C-180°C).

La condensation des vapeurs de tête de la colonne de raffinat C4 peut ainsi être, pour tout ou partie, réalisée au moyen d'une génération de vapeur basse pression où par échange direct avec le rebouilleur d'une autre colonne.

### Colonne de désorbant (non représentée)

Cette colonne est utilisée lorsque l'unité de séparation du paraxylène est du type adsorption du paraxylène. Une petite portion du désorbant circulant dans l'unité d'adsorption du paraxylène P2 est envoyée vers la colonne de désorbant (non représentée) de manière à en éliminer les composés lourds qui sinon s'accumuleraient dans la boucle.

### Unité d'isomérisation des xylènes P3

L'unité d'isomérisation des xylènes P3 est utilisée pour convertir une charge appauvrie en paraxylène en un flux de xylènes à l'équilibre thermodynamique (noté isomérat).

Tout type de catalyseur susceptible d'isomériser les hydrocarbures à 8 atomes de carbones peut être utilisé dans le procédé et le dispositif de séparation selon la présente invention. De préférence, on utilise un catalyseur contenant un métal déshydrogénant comme le platine, le palladium ou le nickel et une phase acide, par exemple une alumine dopée, une zéolithe telle que la mordénite, la MFI, la zéolithe Y, ou les tamis moléculaires zéolitiques ou non zéolithiques comportant une acidité tels que les alumino phosphates (e.g. aluminophosphates AIPO, silicoaluminophosphates SAPO). On peut ainsi de manière plus préférée utiliser un catalyseur d'isomérisation comprenant une zéolithe de type structural EUO, telle que la zéolithe EUI, la zéolithe ZSM 50 ou la zéolithe TPZ3 telles que décrites dans les brevets US 4,640,829, EP-B-042226 ou EP- B-051318.

### Déheptaniseur C7

L'effluent de l'unité d'isomérisation des xylènes P3 est envoyé via la ligne 42 au déheptaniseur C7 qui sépare l'isomère (composés C8+ aromatiques) d'une coupe légère en C7- récupérée en tête de ladite colonne déheptaniseur C7. Cette coupe C7- est envoyée via la ligne 71 vers le stripeur C8 pour séparer les composés légers de la coupe C7.

La coupe C8+, formée des xylènes et des composés plus lourds, récupérée en fond du déheptaniseur C7 est recyclée via la ligne 72 vers la colonne des xylènes C2.

Selon un ou plusieurs modes de réalisation, le déheptaniseur C7 est opéré sous vide de façon à avoir une température inférieure à 180°C (e.g. < 165°C) au rebouilleur 100D. Selon un ou plusieurs modes de réalisation, le déheptaniseur C7 est opéré sous vide de façon à avoir une température inférieure à 150°C au rebouilleur 100D. Ainsi, le déheptaniseur C7 peut être rebouilli par de l'énergie basse température (<180°C).

Selon un ou plusieurs modes de réalisation, le produit de tête (ligne 71) du déheptaniseur C7 opéré sous vide est alors dirigé vers le stripeur C8 comportant en tête de colonne un flux vapeur (ligne 80), constitué d'incondensables, purgé en dehors du procédé.

Selon un ou plusieurs modes de réalisation, compte tenu de la teneur en légers (C4-) possiblement importante dans le déheptaniseur (C7), le produit de tête du déheptaniseur C7, peut comprendre une phase vapeur 70 et une phase liquide 71 (toutes deux issues du ballon de reflux).

### Stripeur C8

Le stripeur (ou colonne de stripage) C8 est alimenté par la tête du déheptaniseur C7.

La coupe C7- stabilisée est récupérée en fond du stripeur C8 pour être envoyée à l'unité d'extraction des aromatiques P1 via la ligne 81.

Selon un ou plusieurs modes de réalisation, le stripeur C8 est aussi alimenté par le produit de tête de la colonne de reformat C1. Les composés les plus légers, tels que de l'air et/ou de l'oxygène issus de l'opération sous vide de la colonne de reformat C1 peuvent être purgés en tête de la colonne de stripage.

Selon un ou plusieurs modes de réalisation, le stripeur C8 est aussi alimenté par le produit de tête de la colonne de purification C6. Les composés les plus légers, tels que de l'air et/ou de l'oxygène issus de l'opération sous vide de la colonne de purification C6 peuvent être purgés en tête de la colonne de stripage.

Selon un ou plusieurs modes de réalisation, le stripeur C8 comprend un rebouilleur 100E. Les composés légers (C4-) issus de la tête du stripper C8 sont mélangés via la ligne 80 aux composés légers issus de la tête du déheptaniseur C7 et purgés.

### Colonne des aromatiques lourds C3

Les composés aromatiques en C9+ récupérés en fond de la colonne des xylènes C2 sont envoyés via la ligne 21 vers la colonne des aromatiques lourds C3 qui sépare les composés aromatiques en C9 et C10 des composés plus lourds (tels que le naphtalène) qui peuvent avoir un effet défavorable sur le catalyseur de transalkylation et qui sont récupérés en fond via la ligne 31.

Selon un ou plusieurs modes de réalisation, la colonne des aromatiques lourds C3 est opérée à basse pression, dans une gamme de pression comprise entre 0,1 et 0,2 MPa absolus (au ballon de reflux), de manière à minimiser à la fois la température et la quantité de chaleur à apporter au rebouilleur de ladite colonne des aromatiques lourds C3.

### Unité de transalkylation P4

Les composés aromatiques en C9 et C10 récupérés en tête de la colonne des aromatiques lourds C3 sont envoyés via la ligne 30 vers l'unité de transalkylation P4.

En référence à la figure 1, lesdits composés aromatiques en C9 et C10 sont mélangés avec le toluène en provenance du fond de la colonne de benzène C9 (ligne 100).

L'unité de transalkylation P4 convertit le toluène et les composés aromatiques en C9+ provenant de la colonne de reformat C1 et de l'isomérat de l'unité d'isomérisation des xylènes P3 (après passage par la colonne des xylènes C2 et la colonne des aromatiques lourds C3) en un mélange de xylènes et de benzène via une réaction limitée par la thermodynamique.

Tout type de catalyseurs de transalkylation est utilisable dans le procédé et le dispositif de séparation selon la présente invention, par exemple des catalyseurs à base de mordénite ou faujasite décrits dans le brevet US 3,437,710 ou les catalyseurs à base de zéolithes MCM-22 ou béta décrits dans le brevet US 5,030,787, ou les catalyseurs à base de zéolithes mordénite et MFI tels que décrits dans la demande de brevet US2012/0065446. Ces catalyseurs comprennent généralement en sus un composé métallique choisi de préférence parmi le groupe formé par le rhénium, le nickel, le cobalt, le molybdène, le tungstène, le palladium, et le platine.

### Colonne de stabilisation C11

L'effluent de l'unité de transalkylation P4 qui contient du benzène et des xylènes ainsi du toluène et des composés en C9+ non convertis est envoyé via la ligne 102 vers la colonne de stabilisation C11 qui sépare les composés plus légers que le benzène, du benzène et des composés aromatiques plus lourds notés C7+.

Le gaz quittant (le ballon de reflux de) la colonne de stabilisation C11 est envoyé via la ligne 110 en limite du complexe aromatique. Une coupe de benzène non purifié est soutirée latéralement et envoyée via la ligne 111 vers la colonne de stripage C8 qui permet de séparer les composés légers de ladite coupe.

Selon un ou plusieurs modes de réalisation, la condensation partielle des gaz de tête de la colonne de stabilisation C11 est obtenue au moyen d'un aéroréfrigérant éventuellement suivi d'un réfrigérant à eau. Selon un ou plusieurs modes de réalisation, la colonne de stabilisation C11 comprend un rebouilleur 100C.

### Colonne de toluène C10

En référence à la figure 1, la colonne de toluène C10 est alimentée par la coupe C7+ issue du fond de la colonne de stabilisation C11 (ligne 112).

Selon un ou plusieurs modes de réalisation, la coupe C6-C7 issue de l'unité d'extraction des aromatiques P1 est envoyée vers la colonne de toluène C10.

Le produit de tête de la colonne de toluène C10 est une coupe riche en composés C7- (e.g. essentiellement toluène + composés C6-) et le produit de fond une coupe C8+ riche en composés aromatiques à 8 atomes de carbones.

La coupe C8+ extraite en fond de la colonne de toluène C10 (*i.e*., produit de fond de la colonne de toluène C10 enrichi en composés à 8 atomes de carbone ou plus) est recyclée via la ligne 101 vers la colonne des xylènes C2 qui sépare les C9+ et plus lourds de la coupe C8 aromatiques alimentant le dispositif de séparation.

L'effluent issu de la tête de la colonne de purification C6 pouvant être d'un débit très faible par rapport au deux autre flux traités par les colonnes de toluène et de benzène, selon un ou plusieurs modes de réalisation de la présente invention (non représenté), ledit effluent est mélangé avec la coupe C7+ issue du fond de la colonne C11.

Selon un ou plusieurs modes de réalisation, la colonne de toluène C10 est opérée sous vide de façon à avoir une température inférieure à 180°C (e.g. < 165°C) au rebouilleur 100A. Selon un ou plusieurs modes de réalisation, la colonne de toluène C10 est opérée sous vide de façon à avoir une température inférieure à 150°C au rebouilleur 100A. Ainsi, la colonne de toluène C10 peut être rebouillie par de l'énergie basse température (<180°C).

Selon un ou plusieurs modes de réalisation, le produit de tête de la colonne de toluène C10 est dirigé vers la colonne de benzène C9 comportant en tête de colonne un flux vapeur (ligne 92), constitué d'incondensables, purgé en dehors du procédé.

### Colonne de benzène C9

En référence à la figure 1, la coupe C7- extraite en tête de la colonne de toluène C10 (*i.e*., produit de tête de la colonne de toluène enrichi en benzène et en toluène) est dirigée via la ligne 91 vers la colonne de benzène C9.

Selon un ou plusieurs modes de réalisation de la présente invention, par exemple lorsque la coupe C6-C7 issue de l'unité d'extraction des aromatiques P1 contient très peu de C8+ (par exemple <1% poids de C8+ dans la coupe C6-C7, de façon préférée <0,5% poids et encore plus préférée <0,3% poids), la coupe C6-C7 issue de l'unité d'extraction des aromatiques P1 est dirigée vers la colonne de benzène C9 en aval de la colonne de toluène (en mélange ou séparément avec la tête de la colonne de toluène C10). Selon un ou plusieurs modes de réalisation, l'alimentation de la colonne de benzène C9 issue de l'unité d'extraction des aromatiques P1 est effectuée séparément et préférablement au-dessus (en aval) de l'alimentation issue de la tête de la colonne de toluène C10.

A partir de la colonne de benzène C9, le produit de tête enrichi en benzène est extrait en tant que produit final via la ligne 90. Selon un ou plusieurs modes de réalisation, le produit enrichi en benzène est extrait par soutirage latéral. Selon un ou plusieurs modes de réalisation, des composés légers et incondensables, notamment ceux issus de l'entrée d'air liée à l'opération sous vide des colonnes de toluène (C10) et/ou de purification (C6) sont purgés via un flux vapeur en tête de colonne (ligne 92). Le produit de fond de la colonne de benzène C9 enrichi en toluène est dirigé via la ligne 100 vers l'unité de transalkylation P4.

Selon un ou plusieurs modes de réalisation, la colonne de benzène C9 est opérée à basse pression dans une gamme allant de 0,1 à 0,2 MPa absolus (au ballon de reflux) de manière à minimiser à la fois la température et la quantité de chaleur à apporter au rebouilleur 100F. Ainsi, la colonne de benzène C9 peut être rebouillie par de l'énergie basse température (e.g. <180°C).

Dans la figure 1, par soucis de simplification, les sections de reflux, ballon de reflux, condenseur et système de mise sous vide éventuels ne sont pas représentés ; tout moyen connu de condensation (ex : aéroréfrigérant et/ou réfrigérant à eau) peut être utilisé.

### Exemple

Dans l'exemple :
- le produit de tête de la colonne de reformat C1 est envoyé au stripeur C8, d'où les composés résultants d'une entrée d'air dans la colonne C1 seront purgés en tête ;
- le produit de tête de la colonne de toluène C10 est envoyé vers la colonne de benzène C9, d'où les composés résultants d'une entrée d'air dans la colonne C10 seront purgés en tête ; et
- le produit de tête de la colonne de purification C6 est envoyé vers la colonne de benzène C9, d'où les composés résultants d'une entrée d'air dans la colonne C6 seront purgés en tête.

Les compositions de la charge et des produits de la colonne de reformat C1 sont indiquées dans le tableau 1

**Tableau 1**

| | | Charge de C1 | Produit de fond de C1 | Produit de tête de C1 |
|---|---|---|---|---|
| C4- | kg/hr | 0 | 0 | 0 |
| C5 | kg/hr | 4142 | 0 | 4142 |
| Non aromatique C6 | kg/hr | 4570 | 0 | 4570 |
| Aromatique C6 | kg/hr | 9298 | 0 | 9298 |
| Non aromatique C7 | kg/hr | 4042 | 0 | 4042 |
| Aromatique C7 | kg/hr | 44891 | 449 | 44442 |
| Non aromatique C8 | kg/hr | 571 | 27 | 544 |
| Aromatique C8 | kg/hr | 48033 | 47793 | 240 |
| C9+ | kg/hr | 27452 | 27452 | 0 |
| **TOTAL** | **kg/hr** | **142999** | **75721** | **67279** |

Comme indiqué dans le tableau 2, les conditions indiquées précédemment pour que, selon l'invention, la colonne de reformat C1 puisse être opérée sous vide sont remplies :

**Tableau 2**

| | |
|---|---|
| Taux (poids) de récupération des C8+ en fond | 99% |
| Taux (poids) de récupération des C7- en tête | 99,3% |
| Teneur de C8 en fond | 63,2% poids |

Par ailleurs la teneur en léger (C4-) est inférieure à 0,1% poids.
La colonne de reformat C1 opérée à 40°C et 0,05 MPa abs en tête présente une température d'environ 135°C au rebouilleur 20A.

Les compositions de la charge et des produits de la colonne de toluène C10 sont indiquées dans le tableau 3.

**Tableau 3**

| | | Charge de C10 | Produit de fond de C10 | Produit de tête de C10 |
|---|---|---|---|---|
| C4- | kg/hr | 0 | 0 | 0 |
| C5 | kg/hr | 0 | 0 | 0 |
| Non aromatique C6 | kg/hr | 27 | 0 | 27 |
| Aromatique C6 | kg/hr | 12297 | 0 | 12297 |
| Non aromatique C7 | kg/hr | 15 | 0 | 15 |
| Aromatique C7 | kg/hr | 62585 | 259 | 62326 |
| Non aromatique C8 | kg/hr | 1 | 0 | 1 |
| Aromatique C8 | kg/hr | 52083 | 51822 | 260 |
| C9+ | kg/hr | 18682 | 18682 | 0 |
| **TOTAL** | **kg/hr** | **145690** | **70763** | **74926** |

Comme indiqué dans le tableau 4 les conditions indiquées précédemment pour que, selon l'invention, la colonne de toluène C10 puisse être opérée sous vide sont remplies :

**Tableau 4**

| | |
|---|---|
| Taux (poids) de récupération des C8+ en fond | 99,6% |
| Taux (poids) de récupération des C7- en tête | 99,7% |
| Teneur de C8 en fond | 73,2% poids |

Par ailleurs la teneur en léger (C4-) est inférieure à 0,1% poids.
La colonne de toluène C10 opérée à 40°C et 0,05 MPa abs en tête présente une température d'environ 135°C au rebouilleur 100A.

Les compositions de la charge et des produits de la colonne de purification C6 sont indiquées dans le tableau 5.

**Tableau 5**

| | | Charge de C6 | Produit de fond de C6 | Produit de tête de C6 |
|---|---|---|---|---|
| C4- | kg/hr | 0 | 0 | 0 |
| C5 | kg/hr | 0 | 0 | 0 |
| Non aromatique C6 | kg/hr | 0 | 0 | 0 |
| Aromatique C6 | kg/hr | 124 | 0 | 124 |
| Non aromatique C7 | kg/hr | 0 | 0 | 2 |
| aromatique C7 | kg/hr | 1159,5 | 4,5 | 1155,0 |
| Non aromatique C8 | kg/hr | 0 | 0 | 0 |
| Aromatique C8 | kg/hr | 90361 | 90335 | 26 |
| C9+ | kg/hr | 18 | 18 | 0 |
| **TOTAL** | **kg/hr** | **91662,5** | **90357,5** | **1307,0** |

Comme indiqué dans le tableau 6, les conditions indiquées précédemment pour que, selon l'invention, la colonne de purification C6 puisse être opérée sous vide sont remplies :

**Tableau 6**

| | |
|---|---|
| Taux (poids) de récupération des C8+ en fond | 99,97% |
| Taux (poids) de récupération des C7- en tête | 99,8% |
| Teneur de C8 en fond | 99,98% poids |

Par ailleurs la teneur en léger (C4-) est inférieure à 0,1% poids.
La colonne de purification C6 opérée à 40°C et 0,06 MPa abs en tête présente une température d'environ 130°C au rebouilleur 100B.

L'opération sous vide de ces trois colonnes à distiller (C1, C10 et C6) permet à l'échelle du complexe une réduction de la consommation électrique de 46% par rapport aux procédés et dispositifs de référence. La réduction de consommation électrique étant notamment due au fait qu'il n'est plus nécessaire de recomprimer la vapeur basse pression issue de l'énergie basse température disponible dans le complexe aromatique avant son utilisation pour rebouillir ces trois colonnes à distiller (C1, C10 et C6).

Si l'on applique l'invention qu'à une seule des 3 colonnes on obtient les résultats suivants.
- 15% de gain par rapport aux procédés et dispositifs de référence pour la seule colonne de reformat C1.
- 20% de gain par rapport aux procédés et dispositifs de référence pour la seule colonne de Toluène C10.
- 11% de gain par rapport aux procédés et dispositifs de référence pour la seule colonne de purification C6.

## Revendications

1. Procédé de séparation d'une charge comprenant du benzène et/ou du toluène et des composés à 8 atomes de carbone ou plus, dans un dispositif de séparation comprenant au moins une colonne à distiller dite de reformat (C1), une unité d'extraction des aromatiques (P1), une unité de séparation du paraxylène (P2), une unité d'isomérisation des xylènes (P3) et une unité de transalkylation (P4), les effluents desdites unités étant séparés dans les colonnes à distiller suivantes : colonne de purification (C6), déheptaniseur (C7) et colonne de toluène (C10),
dans lequel on opère sous vide au moins une desdites colonnes à distiller (C1, C6, C7 et C10) de sorte que :
la majorité des composés à 7 atomes de carbones ou moins sont récupérés dans le produit de tête de la colonne à distiller opérée sous vide, et
la majorité des composés à 8 atomes de carbone ou plus sont récupérés dans le produit de fond de la colonne à distiller opérée sous vide.

2. Procédé de séparation selon la revendication 1, dans lequel on opère la colonne à distiller opérée sous vide de sorte que le produit de fond de ladite colonne à distiller opérée sous vide présente une teneur supérieure à 25% poids en composés à 8 atomes de carbones et/ou composés dont la température normale d'ébullition est inférieure à 150°C.

3. Procédé de séparation selon la revendication 1 ou la revendication 2, dans lequel la pression en tête de colonne de la colonne à distiller opérée sous vide est comprise entre 0,03 MPa et 0,095 MPa.

4. Procédé de séparation selon l'une quelconque des revendications précédentes, dans lequel on opère le rebouilleur de la colonne à distiller opérée sous vide à une température inférieure à 180°C.

5. Procédé de séparation selon l'une quelconque des revendications précédentes, dans lequel on opère la colonne à distiller opérée sous vide de sorte que la teneur en C4- dans le produit de tête de la colonne à distiller opérée sous vide est inférieure à 1% mol.

6. Procédé de séparation selon l'une quelconque des revendications précédentes, dans lequel le produit de tête de la colonne à distiller opérée sous vide est dirigé vers une colonne additionnelle comportant en tête de colonne un flux vapeur purgé en dehors du procédé de séparation.

7. Procédé de séparation selon la revendication 6, dans lequel on opère le rebouilleur de la colonne additionnelle à une température inférieure à 180°C.

8. Procédé de séparation selon l'une quelconque des revendications précédentes, dans lequel une chaleur basse température est apportée au rebouilleur de la colonne à distiller opérée sous vide à partir d'énergie basse température disponible dans le dispositif de séparation, soit par échange direct, soit par l'intermédiaire de génération de vapeur d'eau utilisée sans recompression.

9. Dispositif de séparation d'une charge comprenant du benzène et/ou du toluène et des composés à 8 atomes de carbone ou plus, comprenant au moins une colonne à distiller dite de reformat (C1), une unité d'extraction des aromatiques (P1), une unité de séparation du paraxylène (P2), une unité d'isomérisation des xylènes (P3) et une unité de transalkylation (P4), le dispositif de séparation comprenant en outre les colonnes suivantes pour la distillation des effluents desdites unités : colonne de purification (C6), déheptaniseur (C7) et colonne de toluène (C10),
dans lequel au moins une desdites colonnes à distiller (C1, C6, C7 et C10) est adaptée pour être opérée sous vide de sorte que :
la majorité des composés à 7 atomes de carbones ou moins sont récupérés dans le produit de tête de la colonne à distiller opérée sous vide, et
la majorité des composés à 8 atomes de carbone ou plus sont récupérés dans le produit de fond de la colonne à distiller opérée sous vide.

10. Dispositif de séparation selon la revendication 9, dans lequel l'au moins une desdites colonnes à distiller est adaptée de sorte que le produit de fond de la colonne à distiller opérée sous vide ait une teneur supérieure à 25% poids en composés à 8 atomes de carbones et/ou composés dont la température normale d'ébullition est inférieure à 150°C.

11. Dispositif de séparation selon la revendication 9 ou la revendication 10, dans lequel l'au moins une desdites colonnes à distiller est adaptée de sorte que la pression en tête de colonne de la colonne à distiller opérée sous vide soit comprise entre 0,03 MPa et 0,095 MPa.

12. Dispositif de séparation selon l'une quelconque des revendications 9 à 11, dans lequel l'au moins une desdites colonnes à distiller est adaptée de sorte que le rebouilleur de la colonne à distiller opérée sous vide soit opéré à une température inférieure à 180°C.

13. Dispositif de séparation selon l'une quelconque des revendications 9 à 12, dans lequel l'au moins une desdites colonnes à distiller est adaptée de sorte que la teneur en C4- dans le produit de tête de la colonne à distiller opérée sous vide soit inférieure à 1% mol.

14. Dispositif de séparation selon l'une quelconque des revendications 9 à 13 comprenant en outre une colonne additionnelle, en aval de la sortie de tête de la colonne à distiller opérée sous vide, pour purger un flux vapeur en tête de colonne en dehors du dispositif de séparation.

15. Dispositif de séparation selon la revendication 14, dans lequel la colonne additionnelle est adaptée de sorte que le rebouilleur de la colonne additionnelle soit opéré à une température inférieure à 180°C.
